(19) Europäisches Patentamt
European Patent Office
Office européen des brevets

(11) EP 1 870 075 A1

(12) DEMANDE DE BREVET EUROPEEN

(43) Date de publication:
**26.12.2007 Bulletin 2007/52**

(21) Numéro de dépôt: **07109227.4**

(22) Date de dépôt: **30.05.2007**

(51) Int Cl.:
***A61K 8/04*** *(2006.01)* ***A61K 8/25*** *(2006.01)*
***A61K 8/81*** *(2006.01)* ***A61K 8/92*** *(2006.01)*
***A61Q 1/10*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **20.06.2006 FR 0652545**

(71) Demandeur: **L'Oréal**
**75008 Paris (FR)**

(72) Inventeur: **Jacquier, Isabelle**
**94550, CHEVILLY LARUE (FR)**

(74) Mandataire: **Duvert, Sandra**
**L'OREAL**
**25-29 Quai Aulagnier**
**92665 Asnières-sur-Seine (FR)**

# (54) Mascara comprenant au moins 21% de cire et une charge

(57) La présente invention a pour objet une composition de revêtement des fibres kératiniques comprenant une phase aqueuse, au moins une charge, au moins un polymère filmogène sous forme de particules solides dispersées dans la phase aqueuse, au moins une cire telle que la teneur totale en cire(s) représente au moins 21% en poids par rapport au poids total de la composition, ladite composition présentant une viscosité à 25°C inférieure ou égale à 13,5 Pa.s.

EP 1 870 075 A1

## Description

La présente invention a pour objet une composition de revêtement des fibres kératiniques telles que cils, les sourcils, les cheveux.
Elle se présente en particulier sous la forme d'un mascara, d'un produit pour les sourcils. Plus spécialement, l'invention porte sur un mascara.
Par mascara, on entend une composition destinée à être appliquée sur les fibres kératiniques : il peut s'agir d'une composition de maquillage des fibres kératiniques, une base de maquillage des fibres kératiniques, une composition à appliquer sur un mascara, dite encore top-coat, ou bien encore une composition de traitement cosmétique des fibres kératiniques. Le mascara est plus particulièrement destiné aux fibres kératiniques d'êtres humains, mais également aux faux-cils.
D'une manière générale, les compositions de maquillage des fibres kératiniques sont constituées d'au moins une cire ou d'un mélange de cires dispersé dans une phase aqueuse.
C'est en particulier à travers la quantité de cire, qui permet de structurer la composition, que sont ajustées les spécificités d'application recherchées pour les compositions, comme par exemple leur fluidité ou consistance, leur pouvoir couvrant et/ou leur pouvoir recourbant, ainsi que leur pouvoir épaississant (encore appelé pouvoir chargeant ou maquillant).
Ce type de composition présentant parfois une tenue insuffisante sur les cils, il est connu d'utiliser dans ces compositions polymères filmogènes sous forme de dispersion de particules solides dans une phase aqueuse (ou latex).
Toutefois, l'utilisation de latex peut entraîner un épaississement de la composition ainsi qu'une sensation de collant à l'application, qui ne permet pas l'obtention d'un dépôt lisse et homogène sur les cils.
Il est donc difficile d'obtenir une composition de maquillage des fibres kératiniques comprenant une forte teneur en solides et donc un effet volumateur satisfaisant tout en présentant une application facile et homogène et une bonne tenue sur les fibres kératiniques.
L'invention a pour objet une composition de revêtement des fibres kératiniques comprenant une phase aqueuse, au moins une charge en une teneur d'au moins 0,1% en poids par rapport au poids total de la composition, au moins un polymère filmogène sous forme de particules solides dispersées dans la phase aqueuse, au moins une cire telle que la teneur totale en cire(s) représente au moins 21% en poids par rapport au poids total de la composition, ladite composition présentant une viscosité à 25°C inférieure ou égale à 13,5 Pa.s.
La composition selon l'invention comprend bien entendu un milieu cosmétiquement acceptable, c'est-à-dire un milieu non toxique et susceptible d'être appliqué sur les matières kératiniques d'êtres humains et d'aspect, d'odeur et de toucher agréables.
La présente invention vise également un procédé de soin ou de maquillage des fibres kératiniques, caractérisé par le fait que l'on applique sur lesdites fibres une composition conforme à l'invention.
Elle se rapporte en outre à l'utilisation d'une composition conforme à l'invention pour obtenir un maquillage chargeant des fibres kératiniques, un dépôt lisse et homogène et/ou de bonne tenue sur les fibres kératiniques.
Au sens de la présente invention, on entend qualifier par le terme « chargeant » la notion d'un maquillage épais et volumateur des fibres kératiniques, en particulier des cils.
De préférence, la composition selon l'invention est une composition non rincée.

<u>Viscosité</u>

La viscosité de la composition est mesurée à 25°C à l'aide d'un Rhéomat 180 (Société LAMY) équipé d'un mobile MS-R1, MS-R2, MS-R3, MS-R4 ou MS-R5 choisi en fonction de la consistance de la composition, tournant à un vitesse de rotation de 200 t.min-1. La mesure est prise après 10 min de rotation. Les mesures de viscosité sont réalisées au maximum 1 semaine après fabrication.
La composition selon l'invention présente une viscosité inférieure ou égale à 13,5 Pa.s, allant par exemple de 8 à 13,5 Pa.s, de préférence de 9 à 13 Pa.s, mieux de 9 à 12 Pa.s.

<u>Cire(s)</u>

La cire considérée dans le cadre de la présente invention est d'une manière générale un composé lipophile, solide à température ambiante (25 °C), déformable ou non, à changement d'état solide/liquide réversible, ayant un point de fusion supérieur ou égal à 30 °C pouvant aller jusqu'à 100 °C et notamment jusqu'à 90 °C.
En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
En particulier, les cires convenant à l'invention peuvent présenter un point de fusion supérieur ou égal à 45 °C, et en particulier supérieur ou égal à 55°C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.
Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.
Les cires susceptibles d'être utilisées dans les compositions selon l'invention sont choisies parmi les cires, solides, à température ambiante d'origine animale, végétale, minérale ou de synthèse et leurs mélanges.
Les cires pouvant être utilisées dans les compositions selon l'invention présentent généralement une dureté allant de 0,01 MPa à 15 MPa, notamment supérieure à 0,05 MPa et en particulier supérieure à 0,1 MPa.
La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.
Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.
La valeur de la dureté est la force de compression maximale mesurée divisée par la surface du cylindre du texturomètre en contact avec la cire.

A titre illustratif des cires convenant à l'invention, on peut notamment citer les cires hydrocarbonées comme la cire d'abeille, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.
On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.
On peut encore citer les cires de silicone, les cires fluorées.
On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.
Selon un mode de réalisation préféré, les compositions selon l'invention comprennent au moins une cire dite cire collante c'est-à-dire possédant un collant supérieur ou égal à 0,1 N.s et une dureté inférieure ou égale à 3,5 MPa. La cire collante utilisée peut posséder notamment un collant allant de 0,1 N.s à 10 N.s, en, particulier allant de 0,1 N.s à 5 N.s, de préférence allant de 0,2 à 5 N.s et mieux allant de 0,3 à 2 N.s.
Le collant de la cire est déterminé par la mesure de l'évolution de la force (force de compression) en fonction du temps, à 20 °C selon le protocole indiqué précedemment pour la dureté.
Pendant le temps de relaxation de 1 s, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force. La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange.
Une telle cire est notamment vendue sous les dénominations « Kester Wax K 82 P® », « Hydroxypolyester K 82 P® » et « Kester Wax K 80 P® » par la société KOSTER KEUNEN.

Dans la présente invention, on peut également utiliser des cires fournies sous forme de petites particules ayant une dimension exprimée en diamètre « effectif » moyen en volume D[4,3] de l'ordre de 0,5 à 30 micromètres, en particulier de 1 à 20 micromètres, et plus particulièrement de 5 à 10 micromètres, désignées par la suite par l'expression « micro cires ».
Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La composition est caractérisée par son diamètre "effectif" moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.
Les mesures sont réalisées à 25 °C, sur une dispersion de particules diluée, obtenue à partir de la composition de la manière suivante: 1) dilution d'un facteur 100 avec de l'eau, 2) homogénéisation de la solution, 3) repos de la solution durant 18 heures, 4) récupération du surnageant homogène blanchâtre.
Le diamètre « effectif » est obtenu en prenant un indice de réfraction de 1,33 pour l'eau et un indice de réfraction moyen de 1,42 pour les particules.
Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

La composition selon l'invention présente une teneur en cires supérieure ou égale à 21% en poids par rapport au poids total de la composition, de préférence supérieure ou égale à 22% en poids, pouvant aller jusqu'à 40% en poids, mieux jusqu'à 35% en poids, de préférence jusqu'à 30% en poids.

<u>Charges</u>

Les charges peuvent être minérales ou organiques, lamellaires ou sphériques.
Les charges sont présentes en une teneur d'au moins 0,1% en poids en poids par rapport au poids total de la composition, par exemple allant de 0,1 à 25 % en poids, de préférence au moins 0,5 en poids, allant par exemple de 0,5 à 25% en poids, de préférence au moins 1% en poids, en particulier de 1 à 20 % en poids.

Généralement, les charges utilisées selon l'invention sont incolores ou blanches à savoir non pigmentaires,

c'est-à-dire qu'elles ne sont pas utilisées pour conférer une couleur ou teinte particulière à la composition selon l'invention, même si leur utilisation peut conduire de manière inhérente à un tel résultat.
Elles sont donc, à ce titre, distinctes des nacres, des matières pigmentaires organiques comme par exemple le noir de carbone, les pigments de type D & C, et les laques à base de carmin de cochenille, de baryum, strontium, calcium, aluminium et des matières pigmentaires inorganiques comme par exemple le dioxyde de titane, les oxydes de zirconium ou de cérium, ainsi que les oxydes de fer (noir, jaune ou rouge) ou de chrome, le violet de manganèse, le bleu outremer, l'hydrate de chrome et le bleu ferrique qui, elles, sont utilisées pour procurer un effet de teinte et de coloration aux compositions les incorporant.
De tels composés ne sont pas couverts, au sens de l'invention, par la définition de charges non pigmentaires.
Les charges selon l'invention peuvent être ou non enrobées superficiellement, en particulier traitées en surface par des silicones, des acides aminés, des dérivés fluorés ou toute autre substance favorisant la dispersion et la compatibilité de la charge dans la composition.

Selon un mode de réalisation, la composition selon l'invention comprend au moins une charge minérale et au moins une charge organique.

Parmi les charges minérales non pigmentaires utilisables dans les compositions selon l'invention, on peut citer le talc, le mica, la silice, le kaolin, l'amidon, l'hydroxyapatite, le nitrure de bore, les microsphères de silice creuses (Silce Beads de Maprecos), les microcapsules de verre ou de céramique, et leurs mélanges.

Parmi les charges organiques, on peut citer la lauryol lysine, les poudres de polyamide (Nylon® Orgasol de chez Atochem), de poly-b-alanine, de polyéthylène, la lauroy-lysine, l'amidon, les poudres de polymères de tétrafluoroéthylène (telles que le Téflon), les micro sphères creuses polymériques expansées telles que celles de chlorure de polyvinylidène/acrylonitrile comme celles commercialisées sous la dénomination d'Expancel® par la société Nobel Industrie, les poudres acryliques telles que celles commercialisées sous la dénomination Polytrap® par la société Dow Corning, les copolymères acrylates, le polyméthacrylate de méthyle (PMMA), le stéarate d'oligomère d'acide 12-hydroxystéarique, les microbilles de résine de silicone (Tospearls de Toshiba, par exemple), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, les particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel®007WU par la Société AKZO NOBEL, et leurs mélanges.

Selon un mode de réalisation, la composition cosmétique selon l'invention comprend au moins une charge minérale, de préférence choisie parmi les silices, de préférence des microbilles ou microsphères de silice, et au moins une charge organique, de préférence choisie parmi les poudre de polyéthylène.
Selon un mode de réalisation particulier, la charge minérale est présente à raison de 0,1 à 10 % en poids, en particulier de 0,5 à 5% en poids par rapport au poids total de la composition et la charge organique est présente à raison de 0,05 à 5 % en poids, en particulier de 0,1 à 2% en poids par rapport au poids total de la composition.

<u>Phase aqueuse</u>

La composition selon l'invention comprend un milieu aqueux, constituant une phase aqueuse, qui peut former la phase continue de la composition.

La phase aqueuse de la composition selon l'invention est avantageusement une phase aqueuse continue.
Par composition à phase aqueuse « continue », on entend que la composition présente une conductivité, mesurée à 25°C, supérieure à 23 $\mu$S/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leur mélanges.
La phase aqueuse (eau et éventuellement le solvant miscible à l'eau) peut être présente, en une teneur allant de 1 % à 95 % en poids, par rapport au poids total de la composition, de préférence allant de 3 % à 80 % en poids, et préférentiellement allant de 5 % à 60 % en poids.

Système émulsionnant

Les compositions selon l'invention peuvent contenir des agents tensioactifs émulsionnants présents notamment en une proportion allant de 0,1 à 20 %, et mieux de 0,3 % à 15 % en poids par rapport au poids total de la composition.

Selon l'invention, on utilise généralement un émulsionnant choisi de manière appropriée pour l'obtention d'une émulsion huile-dans-eau. En particulier, on peut utiliser un émulsionnant possédant à 25 °C une balance HLB (hydrophile-lipophile balance) au sens de GRIFFIN, supérieure ou égale à 8.
La valeur HLB selon GRIFFIN est définie dans J. Soc. Cosm. Chem. 1954 (volume 5), pages 249-256.
Ces agents tensioactifs peuvent être choisis parmi des agents tensioactifs non ioniques, anioniques, cationiques, amphotériques ou encore des émulsionnants tensioactifs. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.
Les tensioactifs utilisés préférentiellement dans la composition selon l'invention sont choisis parmi :

a) les agents tensioactifs non ioniques de HLB supérieur ou égal à 8 à 25 °C, utilisés seuls ou en mélange; on peut citer notamment :

les éthers oxyéthylénés et/ou oxypropylénés (pouvant comporter de 1 à 150 groupes oxyéthylénés et/ou oxypropylénés) de glycérol ;
les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool stéarylique à 20 groupes oxyéthylénés (nom CTFA "Steareth-20 ") tel que le BRIJ 78 commercialisé par la société UNIQUEMA, l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination NEODOL 25-7® par SHELL CHEMICALS,
les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P® par la société ICI UNIQUEMA,
les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM® par la société SEPPIC ; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,
les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination Tween 60® par la société UNIQUEMA,
la diméthicone copolyol, telle que celle vendue sous la dénomination Q2-5220® par la société DOW CORNING,
la diméthicone copolyol benzoate (FINSOLV SLB 101® et 201® de la société FINTEX),
les copolymères d'oxyde propylène et d'oxyde d'éthylène, également appelés polycondensats OE/OP,
et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2CH_2)_a\text{-}OH,$$

formule dans laquelle a va de 2 à 120, et b va de 1 à 100.
Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble

est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC® comme les SYNPERONIC PE/ L44® et SYNPERONIC PE/F127® par la société ICI.
b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus tels que :

les esters et éthers d'oses tels que les stéarate de sucrose, cocoate de sucrose, stéarate de sorbitan et leurs mélanges comme l'Arlatone 2121® commercialisé par la société ICI ou le SPAN 65V de la société UNIQUEMA ;
les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
les éthers oxyéthylénés et/ou oxypropylénés tels que l'éther oxyéthyléné de l'alcool stéarylique à 2 groupes oxyéthylénés (nom CTFA "Steareth-2 ") tel que le BRIJ 72 commercialisé par la société UNIQUEMA ;
le mélange de cyclométhicone/diméthicone copolyol vendu sous la dénomination Q2-3225C® par la société DOW CORNING,

c) Les tensioactifs anioniques tels que :

les sels d'acides gras polyoxyéthylénés notamment ceux dérivant des amines ou les sels alcalins, et leurs mélanges ;
les esters phosphoriques et leurs sels tels que le "DEA oleth-10 phosphate" (Crodafos N 10N de la société CRODA) ou le phosphate de monocétyle monopotassique (Amphisol K de Givaudan ou ARLATONE MAP 160K de la société UNIQUEMA);
les sulfosuccinates tels que le "Disodium PEG-5 citrate lauryl sulfosuccinate" et le "Disodium ricinoleamido MEA sulfosuccinate";
les alkyléthersulfates tels que le lauryl éther sulfate de sodium;
les iséthionates ;
les acylglutamates tels que le "Disodium hydrogenated tallow glutamate" (AMISOFT HS-21 R® commercialisé par la société AJINOMOTO) et leurs mélanges.

A titre représentatif des tensioactifs cationiques, on peut notamment citer :

- les alkyl-imidazolidinium tels que l'étho-sulfate d'isostéaryl-éthylimidonium,
- les sels d'ammonium tels que le chlorure de N,N,N-triméthyl-1-docosanaminium (chlorure de Behentrimonium).

Les compositions selon l'invention peuvent également contenir un ou plusieurs tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100® par la société PHOENIX CHEMICAL.

<u>Gélifiant hydrosoluble</u>

La composition selon l'invention peut comprendre un gélifiant hydrosoluble.
Les gélifiants hydrosolubles utilisables dans les compositions selon l'invention peuvent être choisi parmi :

les homo- ou copolymères d'acides acrylique ou méthacrylique ou leurs sels et leurs esters et en particulier les produits vendus sous les dénominations VERSICOL F® ou VERSICOL K® par la société ALLIED COLLOID, ULTRAHOLD 8® par la société CIBA-GEIGY, les acides polyacryliques de type SYNTHALEN K,
les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL,
les copolymères acide polyacryliques/acrylates d'alkyle de type PEMULEN,
l'AMPS (Acide polyacrylamidométhyl propane sulfonique neutralisé partiellement à l'ammoniaque et hautement réticulé) commercialisé par la société CLARIANT,
les copolymères AMPS/acrylamide de type SEPIGEL® ou SIMULGEL® commercialisés par la société SEPPIC, et

les copolymères AMPS/méthacrylates d'alkyle polyoxyéthylénés (réticulés ou non),
les protéines comme les protéines d'origine végétale telles que les protéines de blé, de soja ; les protéines d'origine animale tels que les kératines, par exemples les hydrolysats de kératine et les kératines sulfoniques ;
les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
les polymères ou copolymères acryliques, tels que les polyacrylates ou les polyméthacrylates ;
les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
les polymères d'origine naturelle, éventuellement modifiés, tels que :

les gommes arabiques, la gomme de guar, les dérivés du xanthane, la gomme de karaya ;
les alginates et les carraghénanes;
les glycoaminoglycanes, l'acide hyaluronique et ses dérivés;
la résine shellac, la gomme de sandaraque, les dammars, les élémis, les copals;
l'acide désoxyribonucléïque;
les muccopolysaccharides tels les chondroïtines sulfate,
et leurs mélanges.

Certains de ces gélifiants hydrosolubles peuvent également jouer le rôle de polymères filmogènes.
Le polymère gélifiant hydrosoluble peut être présent dans la composition selon l'invention en une teneur en matières sèches allant de 0,01 % à 60 % en poids, de préférence de 0,5 % à 40 % en poids, mieux de 1 % à 30 % en poids, voire de 5 à 20 % en poids par rapport au poids total de la composition.

Polymère filmogène

La composition selon l'invention comprend avantageusement au moins un polymère filmogène.
Le polymère filmogène peut être présent dans la composition selon l'invention en une teneur en matières sèches (ou matières actives) allant de 0,1 % à 30 % en poids par rapport au poids total de la composition, de préférence de 0,5 % à 20 % en poids, et mieux de 1 % à 15 % en poids.
De préférence, la composition comprend au moins 2% en poids en matières sèches de polymère filmogène, de préférence au moins 3% en poids.
Dans la présente invention, on entend par « polymère filmogène », un polymère apte à former à lui seul ou en présence d'un agent auxiliaire de filmification, un film macroscopiquement continu et adhérent sur les fibres kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.
Parmi les polymères filmogènes utilisables dans la composition de la présente invention, on peut citer les polymères synthétiques, de type radicalaire ou de type polycondensat, les polymères d'origine naturelle, et leurs mélanges.
Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.
Les polymères filmogènes vinyliques peuvent résulter de la polymérisation de monomères à insaturation éthylénique ayant au moins un groupement acide et/ou des esters de ces monomères acides et/ou des amides de ces monomères acides.
Comme monomère porteur de groupement acide, on peut utiliser des acides carboxyliques insaturés $\alpha$, $\beta$ -éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.
Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.
Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.
Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.
Parmi les polycondensats filmogènes, on peut citer les polyuréthanes, les polyesters, les polyesters amides, les polyamides, et les résines époxyesters, les polyurées.
Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.
Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.
Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.
Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+ $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.
Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.
Les polymères d'origine naturelle, éventuellement modifiés, peuvent être choisis parmi la résine shellac, la gomme de sandaraque, les dammars, les élémis, les copals, les polymères cellulosiques, et leurs mélanges.

Le polymère filmogène est avantageusement présent dans la composition sous la forme de particules solides en dispersion dans la phase aqueuse, connue généralement sous le nom de latex ou pseudolatex. Les techniques de préparation de ces dispersions sont bien connues de l'homme du métier.
Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer, Allianz OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous

les dénominations Neorez R-981® et Neorez R-974® par la société AVECIA-NEORESINS, les Avalure UR-405®, Avalure UR-410®, Avalure UR-425®, Avalure UR-450®, Sancure 875®, Sancure 861®, Sancure 878® et Sancure 2060® par la société GOODRICH, Impranil 85® par la société BAYER, Aquamere H-1511® par la société HYDROMER; les sulfopolyesters vendus sous le nom de marque Eastman AQ® par la société Eastman Chemical Products, les dispersions vinyliques comme le Mexomère PAM® de la société CHIMEX et leurs mélanges.

Selon un mode de réalisation avantageux, la composition selon l'invention comprend au moins un polymère filmogène acrylique sous forme de particules solides en dispersion dans la phase aqueuse, ledit polymère résultant de préférence de la polymérisation d'au moins un monomère à insaturation éthylénique choisis parmi les acides carboxyliques $\alpha$, $\beta$ - éthyléniques, leurs esters et leurs amides.

Comme acide carboxylique insaturé $\alpha$,$\beta$-éthyléniques, on peut utiliser l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de ces acides carboxyliques peuvent être choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Il est possible bien entendu d'employer un mélange de ces monomères.

Les esters de l'acide (méth)acrylique particulièrement préférés sont les (méth)acrylates d'alkyle.

Le groupement alkyle des esters peut être soit fluoré, soit perfluoré, c'est-à-dire qu'une partie ou la totalité des atomes d'hydrogène du groupement alkyle sont substitués par des atomes de fluor.

Comme amides desdits acides caboxyliques, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Le polymère filmogène acrylique utilisable selon l'invention peut comprendre, en plus des monomères cités précédement, au moins un monomère styrénique, tel que le styrène ou l'alpha-méthyl styrène.

Comme polymère acrylique, on peut utiliser ceux vendus sous les dénominations "SYNTRAN® 5190 », « SYNTRAN® 5760 », « SYNTRAN ®5009» par la société INERPOLYMER, « DOW LATEX 424® » par la société DOW CHEMICAL.

La composition selon l'invention peut également comprendre un polymère filmogène dit additionnel qui peut être un polymère hydrosouble solubilisé dans la phase aqueuse de la composition.

Le polymère filmogène est présent dans la composition sous la forme de particules en dispersion dans une phase aqueuse ou dans une phase solvant non aqueuse, connue

Selon une autre variante de réalisation de la composition selon l'invention, le polymère filmogène additionnel peut être un polymère solubilisé dans une phase grasse liquide comprenant des huiles ou solvants organiques tels que ceux décrits précédemment (on dit alors que le polymère filmogène est un polymère liposoluble) ou peut être présent dans la composition sous la forme de particules en dispersion dans une phase solvant non aqueuse. De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de

vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth) acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20.

A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polymethylsilsesquioxanes commercialement disponibles, on peut citer celles qui sont commercialisés :

par la société Wacker sous la référence Resin MK tels que la Belsil PMS MK :

par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'un résine de silicone, telle que celles décrite plus haut, et d'un diorganosiloxane tels que décrits dans le document WO 2004/073626.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1411069 ou WO04/028488.

Comme exemples de dispersions non aqueuses de polymère filmogène, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Huiles

La composition peut comprendre une ou plusieurs huiles. Par huile, on entend un corps gras non aqueux liquide à température ambiante (25°C) et pression atmosphérique (760 mm de Hg).

L'huile peut être choisie parmi les huiles volatiles et/ou les huiles non volatiles, et leurs mélanges.

La ou les huiles peuvent être présentes dans la composition selon l'invention en une teneur allant de 0,1 % à 30 % en poids, de préférence de 1% à 20 % en poids par rapport au poids total de la composition.

Par " huile volatile", on entend au sens de l'invention une huile susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg).

Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes (8 $10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale (I) :

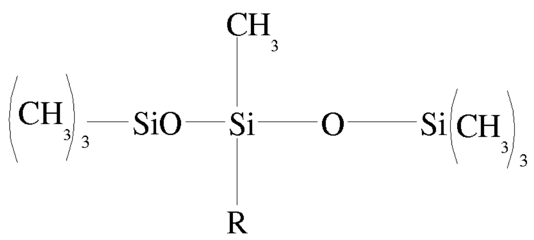

où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des solvants volatils fluorés tels que le nonafluorométhoxybutane ou le perfluorométhylcyclopentane.

La composition peut également comprendre au moins une huile non volatile, et notamment choisie parmi les

huiles hydrocarbonées et/ou siliconées et/ou fluorées non volatiles.
Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit $\geq$ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol; les esters hydroxylés comme le lactate d'isostéaryle, le malate de diisostéaryle; et les esters du pentaérythritol;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- les carbonates,
- les acétales,
- les citrates,
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;
Les huiles fluorées utilisables dans l'invention sont notamment des huiles fluorosiliconées, des polyéthers fluorés, des silicones fluorées telles que décrit dans le document EP-A-847752.

Agent siliconé

La composition selon l'invention comprend avantageusement un agent siliconé qui peut être une silicone oxyalkylénée.
Les silicones oxyalkylénées sont choisies parmi les composés de formule générale (I) appelés aussi diméthicone copolyols :

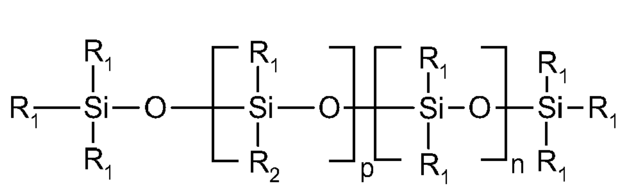 (I)

formule dans laquelle :

- $R_1$, identiques ou différents, représentent un atome d'hydrogène, un radical alkyle linéaire ou ramifié en $C_1$-$C_{30}$ ou un radical phényle,

- $R_2$, identiques ou différents, représentent $-(C_xH_{2x})-(OC_2H_4)_a-(OC_3H_6)_b-OR_3$,
- $R_3$, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 0 à 1000,
- p varie de 1 à 30,
- a varie de 0 à 50,
- b varie de 0 à 50,
- a + b est supérieur ou égal à 1,
- x varie de 1 à 5,
- le poids moléculaire moyen en nombre étant supérieur ou égal à 15000 et de préférence compris entre 25000 et 75000.

De façon préférentielle, on utilise les silicones oxyalkylénées de formule générale (I) qui répondent à au moins une des, et de préférence à toutes les, conditions suivantes :

- $R_1$ désigne le radical méthyle.
- $R_3$ représente un atome d'hydrogène, un radical méthyle ou un radical acétyle, et de préférence hydrogène.
- p varie de 8 à 20.
- a est compris entre 5 et 40 et de préférence entre 15 et 30.
- b est compris entre 5 et 40 et de préférence entre 15 et 30.
- x est égal à 2 ou 3.
- n varie de 20 à 600, de préférence de 50 à 500 et encore plus particulièrement de 100 à 300.

De telles silicones sont par exemple décrites dans le brevet US-4,311,695 qui est inclus à titre de référence.
Des diméthicones copolyols ont en particulier été présentés par la société DOW CORNING lors du 17ème congrès international de l'I.F.S.C.C. d'octobre 1992 et rapportés dans l'article "Water-soluble dimethicone copolyol waxes for personal care industry" de Linda Madore et al., pages 1 à 3.
Ces diméthicones copolyols sont des polydiméthylsiloxanes (PDMS) comportant une ou plusieurs fonctions éthers, solubles dans l'eau (oxyalkylène, notamment oxyéthylène et/ou oxypropylène).
De tels diméthicones copolyols sont notamment vendus par la société GOLDSCHMIDT sous la dénomination ABIL B8851 ou ABIL B88183. On peut citer aussi les composés KF 351 à 354 et KF 615 A vendus par la société SHIN ETSU ou la DMC 6038 de la société WACKER.
Les dérivés de diméthicones copolyols utilisables dans l'invention sont en particulier les diméthicones copolyols à groupement phosphate, sulfate, chlorure de myristamide propyldiméthylammonium, stéarate, amine, glycomodifié, etc.
On peut utiliser comme dérivés de diméthicones copolyols notamment les composés vendus par la société SILTECH sous la dénomination Silphos A100, Siltech amine 65, Silwax WDIS, myristamido silicone quat, ou par la société PHOENIX sous la dénomination Pecosil PS 100.
On peut également utiliser les dérivés vendus par la société WACKER sous la dénomination VP 1661, ou par la société DOW CORNING sous la dénomination 2501 cosmetic wax. On peut encore utiliser les dérivés vendus par la société WACKER sous la dénomination VP 1661, ou par la société DOW CORNING sous la dénomination 2501 cosmetic wax.
Les silicones les plus particulièrement préférées sont par exemple celles vendues par la société DOW CORNING sous la dénomination commerciale Q2-5220 et par la société RHONE POULENC sous la dénomination MIRASIL DMCO.
L'agent siliconé peut représenter de 0,01 à 5 % en poids par rapport au poids total de la composition, de préférence de 0,1 à 2 % en poids.

<u>Matière colorante</u>

La composition selon l'invention peut également comprendre au moins une matière colorante comme les matières pulvérulentes, les colorants liposolubles, les colorants hydrosolubles.
Les matières colorantes pulvérulentes peuvent être choisies parmi les pigments et les nacres.
Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments

nacrés à base d'oxychlorure de bismuth.
Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.
Ces matières colorantes peuvent être présentes en une teneur allant de 0,01 à 30 % en poids par rapport au poids total de la composition.
La composition de l'invention peut comprendre, en outre, tout additif usuellement utilisé en cosmétique tels que les antioxydants, les conservateurs, les fibres, les parfums, les neutralisants, les gélifiants, les épaississants, les vitamines, les agents de coalescence, les plastifiants, et leurs mélanges.

Fibres

La composition selon l'invention peut en outre comprendre des fibres qui permettent une amélioration de l'effet allongeant.
Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.
Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.
En particulier, les fibres ont une longueur allant de 1 μm à 10 mm, en particulier de 0,1 mm à 5 mm et plus particulièrement de 0,3 mm à 3,5 mm. Leur section peut être comprise dans un cercle de diamètre allant de 2 nm à 500 μm, en particulier allant de 100 nm à 100 μm et plus particulièrement de 1 μm à 50 μm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.
Les fibres utilisables dans la composition de l'invention peuvent être choisies parmi les fibres rigides ou non rigides, elles peuvent être d'origine synthétique ou naturelle, minérales ou organiques.
Par ailleurs, les fibres peuvent être traitées ou non en surface, enrobées ou non, colorées ou non colorées.
A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination KERMEL®, KERMEL TECH® par la société RHODIA ou de poly-(p-phénylène-téréphtalamide) (ou d'aramide) notamment vendues sous la dénomination Kevlar ® par la société DUPONT DE NEMOURS.
Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

Actifs cosmétiques

Comme actifs cosmétiques pouvant être utilisés dans les compositions selon l'invention, on peut citer notamment des antioxydants, les conservateurs, les parfums, les neutralisants, émollients, des hydratants, des vitamines et des filtres en particulier solaires.
Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.
La composition selon l'invention peut être conditionnée dans un récipient délimitant au moins un compartiment qui comprend ladite composition, ledit récipient étant fermé par un élément de fermeture.
Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.
L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène. Alternativement, le récipient est réalisé en matériau non thermoplastique, notamment en verre ou en métal (ou alliage).

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.
Le contenu des brevets ou demandes de brevets cités précédemment sont incorporés par référence dans la présente demande.

Les exemples qui suivent sont présentés à titre illustratif et non limitatif de l'invention. Sauf indication contraire, les quantités sont données en gramme.

Exemple 1

On prépare le mascara de composition suivante :

| | |
|---|---|
| - Cire de son de riz | 5,6 |
| - Cire de candelilla | 2 |
| - Cire d'abeille | 5,2 |
| - Cire de carnauba | 6 |
| - Hydroxystearoyl stearate d'alcools gras en C18-C38 (Kester K82 P de Koster keunen) | 4,1 |
| - Poydimethysiloxane oxypropylénée (20 OP)/oxyethylénée (200E) (DC Q2-5220 Resin Modifier de Corning) | 0,5 |
| - Gomme arabique | 2,5 |
| - Hydroxyethyl cellulose | 0,5 |
| - Copolymère Styrène/Acrylates/ammonium methacrylate, à 40% MA dans l'eau avec butylene glycol et sodium laureth-12 sulfate (Syntran 5760 ) | 4 (MA*) |
| - Simethicone | 0,15 |
| - Billes de polyethylene 6-14μm (Micropoly 200L de Micropowders) | 0,5 |
| - Microsphères de silice amorphe 5μm (Sunsphere H51 de ASAHI Glass) | 1 |
| - PEG-40 stearate (Myrj 52P d'UNICHEMA) | 1,5 |
| - Triethanolamine | 2,4 |
| - Acide stearique | 4,6 |
| -D-Panthenol | 0,5 |
| - Ditertiobutyl 4-hydroxytoluene | 0,1 |
| - Oxyde de fer noir | 7 |
| - Conservateurs | qs |
| - Eau | Qsp 100 |
| *MA : matières actives | |

Ce mascara présente un viscosité, mesurée selon la méthode indiquée plus haut, de 10 Pa.s. Il s'applique facilement sur les cils et forme un dépôt volumateur, lisse et homogène, présentant une bonne tenue dans le temps.

### Revendications

1. Composition de revêtement des fibres kératiniques comprenant une phase aqueuse, au moins une charge en une teneur d'au moins 0,1% en poids par rapport au poids total de la composition, au moins un polymère filmogène sous forme de particules solides dispersées dans la phase aqueuse, au moins une cire telle que la teneur totale en

cire(s) représente au moins 21% en poids par rapport au poids total de la composition, ladite composition présentant une viscosité à 25°C inférieure ou égale à 13,5 Pa.s.

2. Composition selon la revendication 1, **caractérisée en ce qu'**elle présente une viscosité allant de 8 à 13,5 Pa.s, de préférence de 9 à 13 Pa.s et mieux de 9 à 12 Pa.s.

3. Composition selon la revendication 1 ou 2, **caractérisée en ce que** la cire est présente en une teneur supérieure ou égale à 22% en poids par rapport au poids total de la composition.

4. Composition selon l'une quelconque des revendications précédentes, **caractérisée en ce qu'**elle comprend au moins une cire dite collante possédant un collant supérieur ou égal à 0,1 N.s et une dureté inférieure ou égale à 3,5 MPa.

5. Composition selon la revendication 4, **caractérisée en ce que** la cire collante est un (hydroxystéaryloxy)stéarate d'alkyle en $C_{20}$-$C_{40}$ (le groupe alkyle comprenant de 20 à 40 atomes de carbone).

6. Composition selon l'une quelconque des revendications précédentes, **caractérisée en ce que** la charge est présente en une teneur d'au moins 0,5% en poids par rapport au poids total de la composition, de préférence au moins 1% en poids.

7. Composition selon l'une quelconque des revendications précédentes, **caractérisée en ce que** la charge représente de 0,1 à 25 %, en particulier de 1 à 20 % en poids par rapport au poids total de la composition.

8. Composition selon l'une quelconque des revendications précédentes, **caractérisée en ce qu'**elle comprend au moins une charge minérale et au moins une charge organique.

9. Composition selon l'une quelconque des revendications précédentes, **caractérisée en ce que** la charge minérale est choisie parmi le talc, le mica, la silice, le kaolin, l'amidon, l'hydroxyapatite, le nitrure de bore, les microsphères de silice creuses, les microcapsules de verre ou de céramique, et leurs mélanges.

10. Composition selon l'une quelconque des revendications précédentes, **caractérisée en ce que** la charge organique est choisie parmi les poudres de polyamide, de poly-b-alanine, de polyéthylène, la lauroy-lysine, l'amidon, les poudres de polymères de tétrafluoroéthylène, les micro sphères creuses polymériques expansées telles que celles de chlorure de polyvinylidène/acrylonitrile, les poudres acryliques, les copolymères acrylates, le polyméthacrylate de méthyle (PMMA), le stéarate d'oligomère d'acide 12-hydroxystéarique, les microbilles de résine de silicone, le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, les particules thermoexpansibles telles que les microsphères non expansées de copolymère de chlorure de vinylidène/d'acrylonitrile/méthacrylate de méthyle ou de copolymère d'homopolymère d'acrylonitrile, et leurs mélanges.

11. Composition selon l'une quelconque des revendications précédentes, **caractérisée en ce qu'**elle comprend au moins une charge minérale choisie parmi les silices , et au moins une charge organique choisie parmi les poudre de polyéthylène.

12. Composition selon l'une des revendications 8 à 11, **caractérisées en ce que** la charge minérale est présente à raison de 0,1 à 10 % en poids, en particulier de 0,5 à 5% en poids par rapport au poids total de la composition et la charge organique est présente à raison de 0,05 à 5 % en poids, en particulier de 0,1 à 2% en poids par rapport au poids total de la composition

13. Composition selon l'une quelconque des revendications précédentes, **caractérisée en ce que** la phase aqueuse est présente en une teneur allant de 1 % à 95 % en poids, par rapport au poids total de la composition, de préférence allant de 3 % à 80 % en poids, et préférentiellement allant de 5 % à 60 % en poids.

14. Composition selon l'une quelconque des revendications précédentes, **caractérisée en ce qu'**elle comprend au moins un polymère filmogène.

15. Composition selon la revendication précédente, **caractérisée en ce que** le polymère filmogène est présent dans

la composition selon l'invention en une teneur en matières sèches allant de 0,1 % à 30 % en poids par rapport au poids total de la composition, de préférence de 0,5 % à 20 % en poids, et mieux de 1 % à 15 % en poids.

**16.** Composition la revendication 14 ou 15, **caractérisée en ce que** la composition comprend au moins 2% en poids en matières sèches de polymère filmogène.

**17.** Composition selon l'une des revendications 14 à 16, **caractérisée en ce que** le polymère filmogène est sous forme de particules solides en dispersion dans la phase aqueuse.

**18.** Composition selon l'une quelconque des revendications précédentes, **caractérisée en ce qu'**elle comprend au moins un polymère filmogène acrylique sous forme de particules solides en dispersion dans la phase aqueuse, ledit polymère résultant de préférence de la polymérisation d'au moins un monomère à insaturation éthylénique choisis parmi les acides carboxyliques $\alpha$,$\beta$-éthyléniques, leurs esters et leurs amides.

**19.** Composition selon la revendication 18, **caractérisée en ce que** l'acide carboxylique insaturé $\alpha$,$\beta$-éthylénique est choisi parmi l'acide acrylique, l'acide méthacrylique, l'acide crotonique, l'acide maléique, l'acide itaconique et leurs mélanges.

**20.** Composition selon la revendication 18, **caractérisée en ce que** l'ester de l'acide caboxylique insaturé $\alpha$, $\beta$-éthylénique est choisi parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .

**21.** Composition selon la revendication 18, **caractérisée en ce que** l'amide de l'acide caboxylique insaturé $\alpha$, $\beta$ -éthylénique, est choisi parmi les N-alkyl ($C_2$-$C_{12}$) (méth)acrylamides, tels que le N-éthyl acrylamide, le N-t-butyl acrylamide, le N-t-octyl acrylamide, le N-undécylacrylamide.

**22.** Composition selon la revendication 18, **caractérisée en ce que** le polymère acrylique comprend au moins un monomère styrénique.

**23.** Composition selon l'une des revendications précédentes, **caractérisée en ce qu'**elle est un mascara.

**24.** Procédé de maquillage des fibres kératiniques, **caractérisé en ce que** l'on applique sur les fibres kératiniques, une composition telle que définie selon l'une quelconque des revendications 1 à 23.

**25.** Utilisation d'une composition selon l'une quelconque des revendications 1 à 23 pour obtenir un maquillage chargeant des fibres kératiniques, un dépôt lisse et homogène et/ou de bonne tenue sur les fibres kératiniques.

Office européen des brevets

# RAPPORT DE RECHERCHE EUROPEENNE

Numéro de la demande
EP 07 10 9227

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | FR 2 844 191 A (OREAL [FR]) 12 mars 2004 (2004-03-12) * page 2, ligne 4 - ligne 21; exemples A-E * * page 6, ligne 19 - page 8, ligne 29 * * page 17, ligne 14 - page 18, ligne 24 * * page 22, ligne 10 - page 23, ligne 23 * ----- | 1-24 | INV. A61K8/04 A61K8/25 A61K8/81 A61K8/92 A61Q1/10 |
| Y | US 2005/061348 A1 (PATEL LILAVATI [US] ET AL) 24 mars 2005 (2005-03-24) * page 1, alinéa 12 - page 2, alinéa 19; exemple 1 * ----- | 1-24 | |
| Y | EP 1 563 825 A (OREAL [FR]) 17 août 2005 (2005-08-17) * page 3, ligne 20 - page 4, ligne 47; exemples 1-3 * ----- | 4,5 | |
| A | FR 2 863 877 A (OREAL [FR]) 24 juin 2005 (2005-06-24) * page 1, ligne 32 - page 2, ligne 36; exemple 1 * * page 3, ligne 20 - page 6, ligne 3 * ----- | 1-24 | DOMAINES TECHNIQUES RECHERCHES (IPC) |
| A | EP 1 064 920 A1 (OREAL [FR]) 3 janvier 2001 (2001-01-03) * page 2, alinéa 2 - page 3, alinéa 18 * * page 14, alinéa 44 - alinéa 55 * * page 19, alinéa 93; exemples 1,2 * ----- | 1-24 | A61K |
| A | EP 1 430 868 A1 (OREAL [FR]) 23 juin 2004 (2004-06-23) * page 7, ligne 34 - page 10, ligne 38; revendications; exemple 4 * * page 14, ligne 51 - page 15, ligne 12 * ----- | 1-24 | |

Le présent rapport a été établi pour toutes les revendications

| Lieu de la recherche | Date d'achèvement de la recherche | Examinateur |
|---|---|---|
| Munich | 21 septembre 2007 | Loloiu, Teodora |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.............................................
& : membre de la même famille, document correspondant

4

EPO FORM 1503 03.82 (P04C02)

## ANNEXE AU RAPPORT DE RECHERCHE EUROPEENNE RELATIF A LA DEMANDE DE BREVET EUROPEEN NO. EP 07 10 9227

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-09-2007

| Document brevet cité au rapport de recherche | Date de publication | Membre(s) de la famille de brevet(s) | Date de publication |
|---|---|---|---|
| FR 2844191 A | 12-03-2004 | AUCUN | |
| US 2005061348 A1 | 24-03-2005 | US 2005061349 A1 | 24-03-2005 |
| EP 1563825 A | 17-08-2005 | FR 2866231 A3 | 19-08-2005 |
| | | JP 2005225881 A | 25-08-2005 |
| | | US 2005215679 A1 | 29-09-2005 |
| FR 2863877 A | 24-06-2005 | AUCUN | |
| EP 1064920 A1 | 03-01-2001 | AT 235878 T | 15-04-2003 |
| | | BR 0006944 A | 31-07-2001 |
| | | CA 2341591 A1 | 11-01-2001 |
| | | CN 1321079 A | 07-11-2001 |
| | | DE 60001874 D1 | 08-05-2003 |
| | | DE 60001874 T2 | 18-12-2003 |
| | | ES 2195843 T3 | 16-12-2003 |
| | | WO 0101936 A1 | 11-01-2001 |
| | | FR 2795634 A1 | 05-01-2001 |
| | | JP 2001031539 A | 06-02-2001 |
| | | MX PA01001630 A | 08-04-2002 |
| | | US 6482400 B1 | 19-11-2002 |
| EP 1430868 A1 | 23-06-2004 | AT 319417 T | 15-03-2006 |
| | | CN 1522683 A | 25-08-2004 |
| | | DE 60303915 T2 | 09-11-2006 |
| | | ES 2262960 T3 | 01-12-2006 |
| | | FR 2848824 A1 | 25-06-2004 |
| | | JP 2004203881 A | 22-07-2004 |
| | | KR 20040055698 A | 26-06-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2792190 A
- FR 2232303 A
- US 5162410 A
- WO 2004073626 A
- EP 1411069 A
- WO 04028488 A
- WO 04055081 A
- EP 847752 A
- US 4311695 A
- US 4887622 A
- FR 2796529
- FR 2761959
- FR 2792618


### Littérature non-brevet citée dans la description


- **VAN DE HULST, H.C.** Light Scattering by Small Particles. Wiley, 1957
- **GRIFFIN.** *J. Soc. Cosm. Chem.,* 1954, vol. 5, 249-256
- la définition des propriétés et des fonctions (émulsionnant) des tensioactifs. Encyclopedia of Chemical Technology, KIRK-OTHMER. WILEY, 1979, vol. 22, 333-432347-377
- **LINDA MADORE et al.** DOW CORNING lors du 17ème congrès international de l'I.F.S.C.C. *Water-soluble dimethicone copolyol waxes for personal care industry,* Octobre 1992, 1-3